(19) Europäisches Patentamt
European Patent Office
Office européen des brevets

(11) EP 0 744 402 B1

(12) **EUROPEAN PATENT SPECIFICATION**

(45) Date of publication and mention of the grant of the patent:
**29.07.1998 Bulletin 1998/31**

(21) Application number: **95907860.1**

(22) Date of filing: **08.02.1995**

(51) Int Cl.[6]: **C07D 417/10**, A61K 31/41

(86) International application number:
**PCT/JP95/00174**

(87) International publication number:
**WO 95/21837 (17.08.1995 Gazette 1995/35)**

# (54) POTASSIUM SALT OF BIPHENYLMETHANE DERIVATIVE AND MEDICINE CONTAINING THE SAME

KALIUMSALZ EINES BIPHENYLMETHANDERIVATES UND DASSELBE ENTHALTENDES MEDIKAMENT

SEL DE POTASSIUM DE DERIVE BIPHENYLMETHANE ET MEDICAMENT LE CONTENANT

(84) Designated Contracting States:
**AT BE CH DE DK ES FR GB GR IE IT LI LU MC NL PT SE**

(30) Priority: **10.02.1994 JP 16220/94**
**10.06.1994 JP 128739/94**

(43) Date of publication of application:
**27.11.1996 Bulletin 1996/48**

(73) Proprietor: **WAKUNAGA SEIYAKU KABUSHIKI KAISHA**
**Osaka-Shi Osaka 532 (JP)**

(72) Inventors:
- **HIRATA, Terukage**
**Wakunaga Seiyaku Kabushiki Kaisha**
**Hiroshima 729-64 (JP)**
- **SAKAE, Nobuya**
**Wakunaga Seiyaku Kabushiki Kaisha**
**Takata-gun Hiroshima 729-64 (JP)**
- **TAMURA, Koichi**
**Wakunaga Seiyaku Kabushiki Kaisha**
**Hiroshima 729-64 (JP)**
- **OKUHIRA,Masayasu**
**Wakunaga Seiyaku Kabushiki Kaisha**
**Takata-gun Hiroshima 7729-64 (JP)**
- **AMANO, Hirotaka**
**Wakunaga Seiyaku Kabushiki Kaisha**
**Hiroshima 729-64 (JP)**
- **YOKOMOTO, Masaharu**
**Wakunaga Seiyaku Kabushiki**
**Takata-gun Hiroshima 729-64 (JP)**
- **NOMIYAMA, Jun**
**Wakunaga Seiyaku Kabushiki Kaisha**
**Takata-gun Hiroshima 729-64 (JP)**

(74) Representative: **Kraus, Walter, Dr. et al**
**Patentanwälte Kraus, Weisert & Partner**
**Thomas-Wimmer-Ring 15**
**80539 München (DE)**

(56) References cited:
**WO-A-93/17681**
**WO-A-94/04516**
**WO-A-96/09301**

EP 0 744 402 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention relates to novel potassium salts of biphenylmethane derivatives having a strong angiotensin II antagonist activity and antihypertensive activity, and also to pharmaceuticals containing the salts.

Angiotensin II is an active center of the renin-angiotensin system, and has powerful vasopressor action and stimulating action for the synthesis and secretion of aldosterone in the adrenal cortex. It is also known to be a substance causing hypertension. Its action is considered to be caused through a specific receptor on various target organs such as adrenal cortex, kidneys, arterioles and the peripheries of sympathetic nerves.

Known conventional examples of substances which shows an antihypertensive effect by pharmacological inhibition of the renin-angiotensin system include angiotensin-converting enzyme inhibitors such as captopril and enarapril, angiotensin II antagonists and renin inhibitors. As angiotensin II antagonist out of these, saralasin ([$Sar^1$, $Ala^8$] AGII), an angiotensin II type peptide, and nonpeptide derivatives such as imidazole derivatives (Japanese Patent Laid-Open Nos. 71073/1981 and 71074/1981, and Japanese Language Laid-Open Publication No. 501020/1991), pyrazole derivatives (Japanese Patent Laid-Open No. 218371/1991) and aminoazole derivatives (W093/17681) are already known.

The peptide derivatives, however, have difficulty in clinical applications because of their short *in vivo* half-life, lack of effectiveness upon oral administration and significant agonistic activities. Among the nonpeptide derivatives, none has been used clinically yet as drugs either.

An object of the present invention is therefore to provide a drug which can be used clinically and has the above-described activities.

With a view toward providing a clinically excellent drug under such circumstances, the present inventors carried out an extensive investigation. As a result, they found that a biphenylmethane derivative represented by the following formula (1) or a salt thereof has an excellent angiotensin II antagonist activity and is useful as a therapeutic for circulatory diseases such as hypertension, heart diseases and cerebral apoplexy and filed a PCT application thereon (W0-A-94/04516).

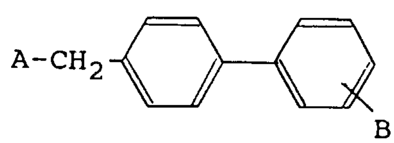

(1)

wherein A represents a group

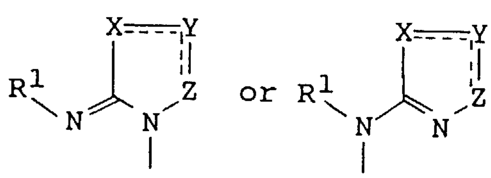

in which $R^1$ represents a hydrogen atom, a lower alkyl group, a lower-cycloalkyl group, a substituted or unsubstituted phenyl group, a substituted or unsubstituted aralkyl group, a substituted or unsubstituted acyl group or an amino acid residue; X represents an oxygen atom, a sulfur atom or a group =CH-, Y represents a nitrogen atom or a group =$CR^2$-, Z represents an oxygen atom, a nitrogen atom or a group =$CR^3$-, said Y and Z not being hetero atoms at the same time, $R^2$ and $R^3$ each independently represents a hydrogen atom, a halogen atom, a substituted or unsubstituted lower alkyl group, a protected or unprotected carboxyl group, a lower-cycloalkyl group, a lower alkenyl group, a lower alkoxyl group, a lower alkylthio group or an aryl group or $R^2$ and $R^3$ may form a substituted or unsubstituted benzene ring together with the adjacent carbon atoms; B represents a cyano group, a protected or unprotected carboxyl group or a protected or unprotected tetrazol-5-yl group and $\overset{\text{---}}{=\!=}$ shows a double bond or a single bond.

As a result of a further investigation, the present inventors have found that among the biphenylmethane derivatives (1) described above, the mono- and di-potassium 2-[[5-ethyl-3-[2'-(1H-tetrazol-5-yl)biphenyl4-yl]methyl-1,3,4-thiadiazolin-2-yliden]aminocarbonyl]1-cyclopentenecarboxylate have a particularly strong angiotensin II antagonist activity and antihypertensive activity and also have extremely high bioavailability in oral administration, leading to the completion of the present invention.

The present invention therefore provides the mono- or di-potassium salt of 2-[[5-ethyl-3-[2'-(1H-tetrazol-5-yl)biphenyl-4-yl]methyl-1,3,4-thiadiazolin-2-yliden]aminocarbonyl]-1-cyclopentenecarboxylate represented by the following formula (2):

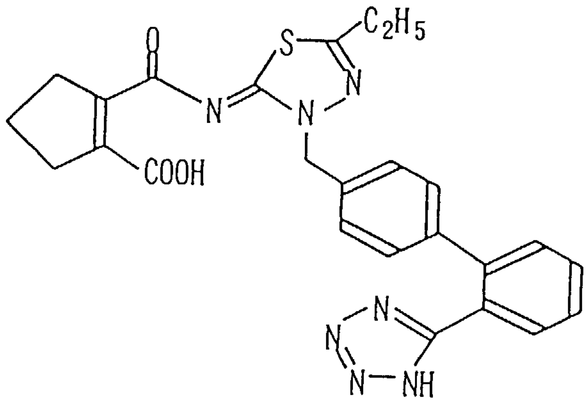

(2)

The present invention also provides a pharmaceutical composition which comprises the mono or dipotassium salt of the compound (2) and a pharmaceutical carrier.

The present invention also provides the mono- or dipotassium salt of the compound (2) for use as a pharmaceutical.

Further, the present invention also provides the mono- or dipotassium salt of the compound (2) for use in a therapeutic method of circulatory diseases, which comprises administering an effective amount of said salt.

Of the mono- and di-potassium salts of the compound (2), said salts pertaining to the present invention, the dipotassium salt is particularly preferred.

The mono- or di-potassium salt of the compound (2), to which salt the present invention is concerned, can exist as different stereoisomers (cis-form, transform). These stereoisomers are also included in the present invention. The compounds of the present invention can also exist as solvated products such as hydrates, which are also included in the present invention.

The mono- or dipotassium salt of the compound (2) can be prepared by various processes. Preferred is the preparation process shown below:

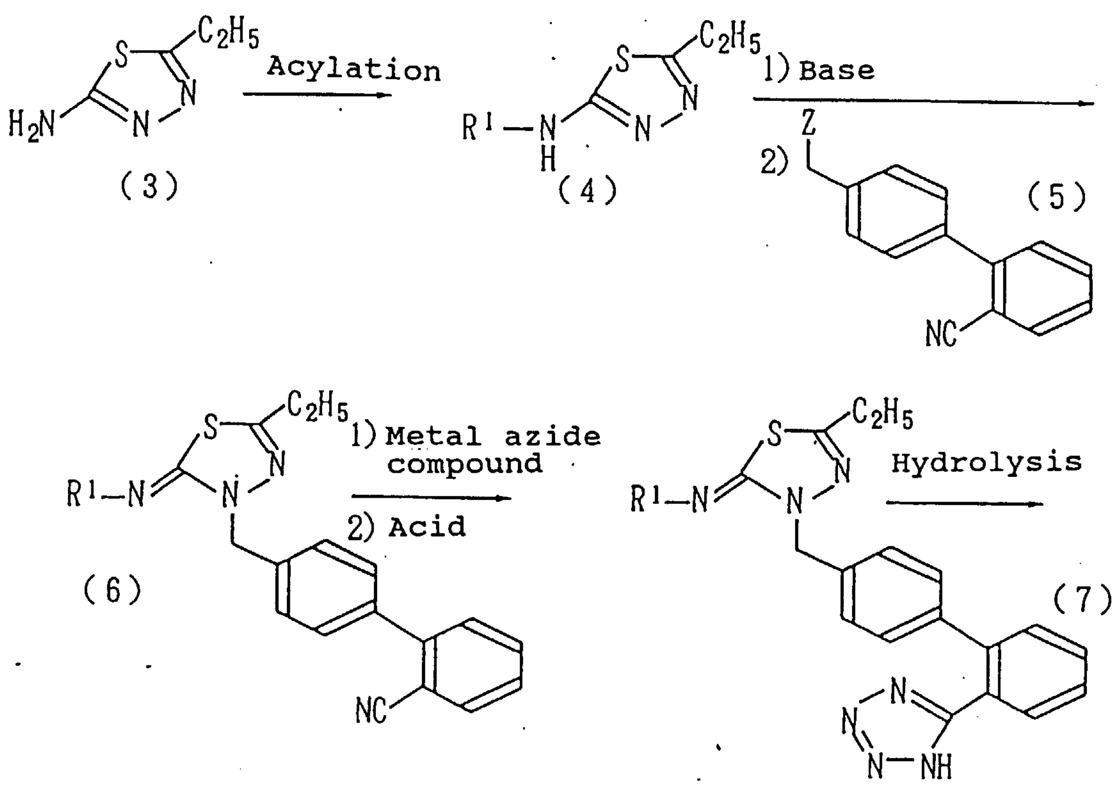


(3) (4) (5) (6) (7)

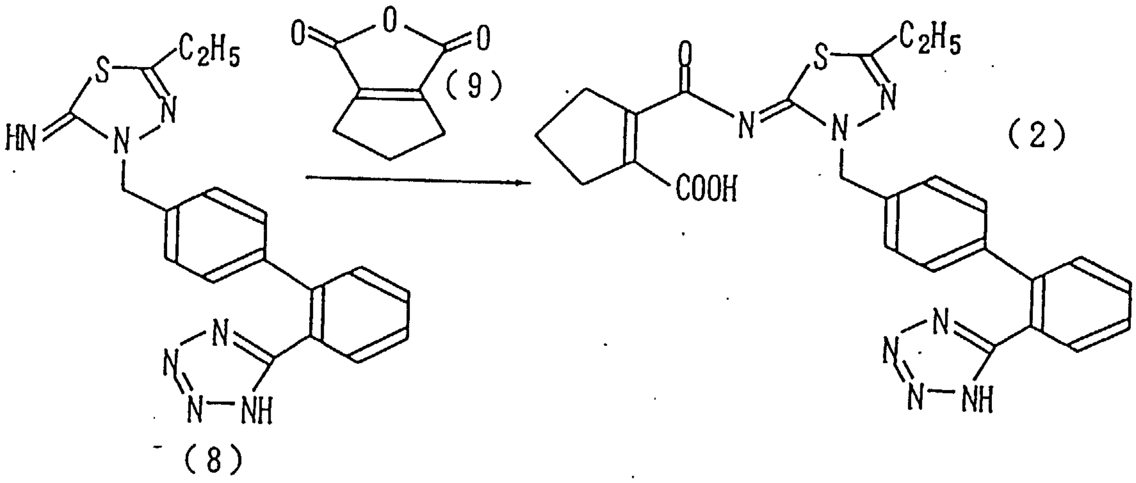

wherein $R^1$ represents a substituted or unsubstituted acyl group and Z represents a halogen atom or a sulfonyloxy group.

Namely, 2-amino-5-ethyl-1,3,4-thiadiazole (3) is subjected to acylation to obtain the compound (4). The compound (4) is condensed with the compound (5) in the presence of a base, whereby the compound (6) is prepared. The compound (6) is then tetrazolylated to obtain the compound (7), followed by hydrolysis to remove the acyl group, whereby the compound (8) is obtained. The compound (8) is then reacted with a compound (9) to obtain the compound (2). The compound (2) so obtained is converted into its mono- or di-potassium salt in a manner known *per se* in the art. The compound of the present invention can be obtained in this way. The compound (7) can also be obtained by reacting the compound (4) with a 4-halogenomethyl-2'-(tetrazol-5-yl)biphenyl.

The above reaction steps will next be described in detail, respectively.

Examples of an acylating agent useful for the acylation of the compound (3) include acetic acid, trifluoroacetic acid or the like. The acylation can be conducted by any desired reaction commonly employed for the acylation of an amino group. Described specifically, the acylation can be conducted by reacting the compound (3) with an acyl chloride or an acid anhydride, which corresponds to a desired acyl group, in an aprotonic polar solvent - such as a halogenated hydrocarbon, e.g., methylene chloride, chloroform, carbon tetrachloride or chlorobenzene; an aromatic hydrocarbon, e.g., benzene or toluene; an ether, e.g., tetrahydrofuran or dioxane; acetonitrile; or N,N-dimethylformamide at temperatures of 0°C to room temperature in the presence or absence of a base such as pyridine, picoline, N,N-dimethylaniline, N-methylmorpholine, dimethylamine, triethylamine, sodium carbonate or potassium carbonate at -70 to 100°C; or by reacting it with an acid such as acetic acid or the acid anhydride thereof at temperatures of room temperature to 150°C.

Examples of the base usable in the above condensation of the compound (4) and compound (5) include sodium hydride, lithium hydride, potassium carbonate, sodium carbonate, sodium alcoholates, potassium t-butoxide, sodium hydroxide, potassium hydroxide, triethylamine and diisopropylethylamine. Any solvent can be used here as long as it does not affect the reaction. Exemplary usable solvents include aprotonic polar solvents such as N,N-dimethylformamide and dimethylsulfoxide; ethers such as diethyl ether, tetrahydrofuran, dioxane, monoglymes and diglymes; halogenated hydrocarbons such as methylene chloride, chloroform and carbon tetrachloride; and alcohols such as methanol, ethanol and propanol.

As a reaction accelerator, a phase transfer catalyst can be added. Examples of the phase transfer catalyst include quaternary ammonium salts such as tetramethylammonium chloride, tetraoctylammonium chloride and tetrabutylammonium bromide; pyridinium salts such as N-neopentyl-4-(N',N'-dimethylamino)pyridium chloride and N-(2-ethylhexyl)-4-(N',N'-dimethylamino)pyridinium chloride; and quaternary phosphonium salts such as tetrabutylphosphonium bromide and tetraphenylphosphonium bromide.

The condensation may ordinarily be conducted at temperatures of -30°C to 150°C, preferably 10°C to 100°C. The reaction time may generally be 10 minutes to 24 hours, preferably 1 hour to 10 hours.

A particularly preferred example of the condensation is the one in which a metal salt of the compound (5) is prepared in an aprotonic polar solvent such as N,N-dimethylformamide by using sodium hydride or potassium-carbonate as a base and then the resulting metal salt is reacted with the compound (4) at temperatures of 0°C to room temperature.

Examples of the halogen atom represented by Z in the compound (5) include a fluorine atom, a chlorine atom, a bromine atom or an iodine atom. Illustrative of the sulfonyloxy group include alkylsulfonyloxy groups such as methanesulfonyloxy, ethanesulfonyloxy and trifluoromethanesulfonyloxy group, and arylsulfonyloxy groups such as benzenesulfonyloxy and p-toluenesulfonyloxy group.

Tetrazolylation of the compound (6) can be conducted in accordance with a known method (Japanese Patent Laid-Open No. 23868/1988), namely, by adding a 1,3 dipolar ring to the compound (6) by using a metal azide compound such as tri-$C_1$-$C_{18}$ alkyltin azide, tri-$C_1$-$C_{18}$ alkylsilyl azide or sodium azide. Specifically, a tetrazol-5-yl derivative (7) can be obtained by adding a metal azide compound to the compound (6) in a solvent such as benzene or toluene, reacting them under heat and then treating the reaction product with hydrochloric acid or the like.

The compound (4) can be prepared in a manner known to date (Japanese Patent Laid-Open No. 23868/1988, 27362/1991 or 74369/1991; J. Org. Chem., 56, 2395-2400(1991).

For the deacylation (deprotection) of the compound (7), any desired known reaction can be employed. For example, the deprotection can be conducted by reacting the compound (7) in an aqueous alkaline solution such as an aqueous sodium hydroxide solution, aqueous potassium hydroxide solution or aqueous sodium carbonate solution or in an acidic solution such as hydrochloric acid or acetic acid at temperatures of room temperature to 100°C, using a solvent miscible with water such as ethanol, methanol, tetrahydrofuran or N,N-dimethylformamide or in a solventless manner.

The reaction between the compound (8) and the compound (9) can be conducted in an aprotonic polar solvent - such as a halogenated hydrocarbon, e.g., methylene chloride, chloroform, carbon tetrachloride or chlorobenzene; an aromatic hydrocarbon, e.g., benzene or toluene; an ether, e.g., tetrahydrofuran or dioxane; acetonitrile; or N,N-dimethylformamide at temperatures of 0°C to room temperature in the presence or absence of a base such as pyridine, picoline, N,N-dimethylaniline, N-methylmorpholine, dimethylamine, triethylamine, sodium car bonate or potassium carbonate at temperatures of -70°C to 100°C.

In the above preparation process, when the tetrazol-5-yl group has a protective group, the protective group can be removed as needed.

For the above deprotection, it is desired to conduct the deprotection by reacting the compound in a water-containing alcohol, or an ether, such as dioxane or tetrahydrofuran, which contains hydrochloric acid, acetic acid or the like, at room temperature or so for approximately 1-10 hours.

The compound (2) so obtained can be converted into its mono- or di-potassium salt in a manner known *per se* in the art. Specifically, it is only necessary to dissolve the compound (2) in a potassium hydroxide solution, followed by precipitation as a salt. It is preferred to employ, as the potassium hydroxide solution, a solution of potassium hydroxide dissolved in an amount at least equivalent to that of the compound (2) in water, methanol, ethanol, n-propyl alcohol, isopropyl alcohol, acetone or the like. The compound (2) is dissolved in the potassium hydroxide solution so obtained. The dissolving temperature can be determined between room temperature and a temperature under heat as desired depending on the compound (2). In addition, a desired method can be chosen for the precipitation of the salt, because some salts precipitate when simply left over but some salts do not precipitate until the solvent is removed to some extent.

The mono- or di-potassium salt of the compound (2), so obtained, can be purified as needed in a manner known *per se* in the art, for example, by dissolving the salt in one or more solvents selected from water, methanol, ethanol, n-propyl alcohol, isopropyl alcohol or acetone and then recrystallizing it therefrom.

When the mono- and di-potassium salts of the compound (2), which pertains to the present invention, are used as therapeutic agents for circulatory diseases, they can be formulated into compositions together with a pharmaceutically-acceptable carrier for parenteral administration such as injection or rectal administration or for oral administration in the form of a solid or a liquid.

Compositions of this invention for use as injections can take the form of pharmaceutically-acceptable germ-free water, non-aqueous solutions, suspensions or emulsions. Exemplary suitable non-aqueous carriers, diluents, solvents and vehicles include propylene glycol, polyethylene glycol, vegetable oils such as olive oil, and injectable organic esters such as ethyl oleate. These preparations can contain one or more auxiliary agents, for example, antiseptics, wetting agents, emulsifiers and dispersants. These formulations can be sterilized, for example, by filtering them through a bacterial filter or by mixing, immediately before use, a sterilizing agent in the form of a germ-free solid composition soluble in sterilized water or one of some other media which can be sterilized and injected.

Exemplary solid preparations for oral administration include capsules, tablets, pills, powders, granules, etc. Upon formulation of these solid preparations, the compounds according to the present invention are generally mixed with at least one inert extender such as sucrose, lactose or starch. One or more materials other than inert extenders, for example, a lubricant such as magnesium stearate can also be incorporated in the preparations upon formulation of the latter in a usual manner. A buffer can also be incorporated in the case of capsules, tablets and pills. Tablets and pills can be applied with an enteric coating.

Illustrative liquid preparations for oral administration include pharmaceutically-acceptable emulsions, solutions, suspensions, syrups and elixirs, which contain an inert diluent employed commonly by those skilled in the art, for example, water. In addition to such an inert diluent, the liquid preparations can also be added with one or more auxiliary agents, for example, wetting agents, emulsifiers, suspending agents, sweetening agents, seasoning agents and perfumes. Preparations for rectal administration are preferred to contain an excipient such as cacao butter or suppository wax in addition to a compound according to the present invention.

The dosage of the mono- or di-potassium salt of the compound (2) according to the present invention depends on

the properties of the compound to be administered, the administration route, the desired treatment term and other factors. It generally ranges from about 0.1 mg/kg to 100 mg/kg per day, with about 0.5-50 mg/kg per day being preferred especially. If desired, this daily dosage can be administered in 2-4 portions.

## Examples

The present invention will hereinafter be described more specifically by examples. It should however be borne in mind that this invention is by no means limited to or by the examples.

### Synthesis Example 1

(1) Synthesis of 2-trifluoroacetamido-5-ethyl-1,3,4-thiadiazole

To a suspension of 200 g of 2-amino-5-ethyl-1,3,4-thiadiazole in 3 L of toluene was added 260 mL of triethylamine at room temperature followed by the addition of 265 mL of trifluoroacetic anhydride under ice cooling. The mixture was stirred for one hour at room temperature. To the reaction mixture was added water and precipitated crystals were collected by filtration. Ethyl acetate was added to the filtrate. The organic layer was separated and dried over anhydrous magnesium sulfate. The solvent was evaporated in vacuo to afford 237.5g (68%) of the title compound.

(2) Synthesis of 4-bromomethyl-2'-cyanobiphenyl

To 110 mL of carbon tetrachloride were added 10.5 g of 4-methyl-2'-cyanobiphenyl, 9.79 g of N-bromosuccinimide and 120 mg of 2,2'-azodiisobutyronitrile. The resulting mixture was refluxed for 2 hours. An insoluble material was removed by filtration under heat. The filtrate was cooled on standing. Precipitated crystals were collected by filtration, whereby 6.6 g (44%) of the title compound was obtained.

(3) Synthesis of 2-trifluoroacetylimino-5-ethyl-3-(2'-cyanobiphenyl-4-yl)methyl-1,3,4-thiadiazoline.

To a suspension of 124.1 g of sodium hydride (55% in oil) in 1.5 L of N,N-dimethylformamide was added 533.7 g of 2-trifluoroacetamido-5-ethyl-1,3,4-thiadiazole obtained in (1) under ice cooling. When evolution of hydrogen ceased, a solution of 643.6 g of 4-bromomethyl-2'-cyanobiphenyl obtained in (2) in 3 L of N,N-dimethylformamide was added dropwise to the reaction mixture. The reaction mixture was stirred for 1 hour at room temperature followed by stirring for 5 hours at 80 °C. The solvent was evaporated in vacuo and to the residue were added water and ethyl acetate. The organic layer was washed with water and dried over anhydrous magnesium sulfate. After evaporation of the solvent in vacuo, the residue was purified by chromatography on silica gel column (eluent; n-hexane:ethyl acetate = 3:1) to afford the crude crystals. The crude crystals were recrystallized from ethanol to give 490.8 g (50%) of the title compound.

(4) Synthesis of 2-trifluoroacetylimino-5-ethyl-3-(2'-cyanobiphenyl-4-yl)methyl1,3,4-thiadiazoline

To 18 L of N,N-dimethylformamide were added 2.44 kg (10.8 mol) of 2-trifluoroacetamido-5-ethyl-1,3,4-thiadiazole and 2.80 kg (10.3 mol) of 4-bromomethyl-2'-cyanobiphenyl followed by the addition of 894 g (6.5 mol) of anhydrous potassium carbonate and 60 g of potassium iodide. The reaction mixture was stirred for 41 hours at room temperature. An insoluble material was removed by filtration and the filtrate was concentrated in vacuo. To the residue were added 45 L of water and 18 L of ethyl acetate. The organic layer was separated and dried over anhydrous magnesium sulfate, and concentrated to half of its initial volume. Precipitated crystals were collected by filtration and washed successively with ethanol and isopropyl ether. The crystals (purity: 95.8%) were recrystallized from 5 L of ethanol to give 2.70 kg of crystals (purity: 94%). This crystals (purity: 94%) were purified by column chromatography on silica gel. (silica gel 10 kg, eluent: n-hexane:ethyl acetate = 3:1→2:1) to afford 2.64 kg (purity: 99%) of the title compound. All the filtrates were combined and the solvent was evaporated. The residue was dissolved in 3 L of hot ethanol and to the solution activated charcoal was added. The mixture was stirred for 30 minutes at 80 °C and filtered through Celite. The filtrate was then allowed to stand overnight at room temperature. Precipitated crystals were collected by filtration. The crystals were treated again with active charcoal as described above, whereby 562 g ( purity: 98%) of the title compound was obtained. The filtrate was evaporated and the residue was purified by column chromatography on silica gel (silica gel 4 kg, eluent; n-hexane:ethyl acetate = 5:1) to afford 371 g of the title compound. In total, 3.57 kg of the title compound was obtained.
Property: Colorless prism crystals
Melting point: 106-107°C
$^1$H-NMR (δppm in $CDC\ell_3$)
7.43-7.79(8H,m), 5.60(2H,s), 2.95(2H,q), 1.39(3H,t)

(5) Synthesis of 2-trifluoroacetylimino-5-ethyl-3-[2'-(1H-tetrazol-5-ylbiphenyl-4-yl]methyl-1,3,4-thiadiazoline

To 1 L of toluene were added 490.8 g of 2-trifluoroacetylimino-5-ethyl-3-(2'-cyanobiphenyl-4-yl)methyl-1,3,4-thiadiazoline obtained in (4) and 485.5 g of trimethyltin azide. The mixture was refluxed for 40 hours. To the reaction mixture was added 200 mL of concentrated hydrochloric acid followed by stirring for 10 minutes. The reaction mixture was extracted with 5 L of ethyl acetate. The organic layer was washed with water and dried over anhydrous magnesium sulfate. The solvent was removed in vacuo to afford the title compound, which was not purified for use in the next step.

(6) Synthesis of 2-imino-5-ethyl-3-[2'-(1H-tetrazol-5-yl)biphenyl-4-yl]methyl-1,3,4-thiadiazoline hydrochloride

To a mixture of 4 L of tetrahydrofuran and 200 mL of water and 2-trifluoroacetylimino-5-ethyl-3-[2'-(1H-tetrazol-5-yl)biphenyl-4-yl]methyl-1,3,4-thiadiazoline obtained in (5) was added 94.4 g of sodium hydroxide, and the mixture was refluxed for 7 hours. The reaction mixture was concentrated in vacuo. To the residue were added water and ethyl acetate. The aqueous layer was separated and acidified with hydrochloric acid. Precipitated crystals were collected by filtration and 168 g of the title compound was obtained. From the ethyl acetate layer, crystals were precipitated and collected by filtration, whereby 80 g of the title compound was further obtained.
Property: Colorless powder
Melting point: 205-206°C
$^1$H-NMR (δppm in DMSO-$d_6$)
7.53-7.73(4H,m), 7.28(2H,d), 7.14(2H,d), 5.43(2H,s), 2.89(2H,q), 1.22(3H,t)

(7) Synthesis of 2-[[5-Ethyl-3-[2'-(1H-tetrazol-5-yl)biphenyl-4-yl]methyl-1,3,4-thiadiazolin-2-ylidene]aminocarbonyl]-1-cyclopentenecarboxylic acid

To 2 mL of N,N-dimethylformamide were added 200 mg of 5-ethyl-2-imino-3-[2'(1H-tetrazol-5-yl)biphenyl-4-yl]methyl-1,3,4-thiadiazoline hydrochloride and 76 mg of 1,2-cyclopentenedicarboxylic anhydride. The reaction mixture was stirred for 2 hours at room temperature and poured into water. Precipitated crystals were collected by filtration, washed with water and ethanol, and then dried, whereby 170 mg of the title compound was obtained.
Property: Colorless crystals
Melting point: 234-235°C
$^1$H-NMR (δppm in $CDC\ell_3$ + $CD_3OD$)
7.76(1H,d), 7.35-7.65(3H,m), 7.29(2H,d), 7.11(2H,d), 5.52(2H,s), 3.03(4H,t), 2.94(2H,q), 1.89(2H,m), 1.39(3H,t)

Example 1

Synthesis of dipotassium 2-[[5-ethyl-3-[2'-(1H-tetrazol-5-yl)biphenyl-4-yl]methyl-1,3,4-thiadiazolin-2-ylidene]aminocarbonyl]-1-cyclopentenecarboxylate (compound A) To a mixture of 400 mL of 0.1 N potassium hydroxide (ethanolic) and 600 mL of ethanol was added 10 g of 2-[[5-ethyl-3-[2'-(1H-tetrazol-5-yl)biphenyl-4-yl]methyl-1,3,4-thiadiazolin-2-ylidene]aminocarbonyl]-1-cyclopentenecarboxylic acid. After the mixture had been completely dissolved by heating on water bath, the solvent was evaporated in vacuo until the volume of solution decreased to about 200 mL as a whole. Precipitated crystals were collected by filtration, washed with ethanol, and then dried in vacuo, whereby 11g of the title compound was obtained.
Melting point: >280°C
IR(KBr)$cm^{-1}$: 1642(-COOK) 1570 (=N-CO-)
$^1$H-NMR(δ ppm in $D_2O$) : 7.51-7.62(3H,m), 7.37
7.39(1H,m), 7.31(2H,d), 7.04(2H,d), 5.49(2H,s), 2.66-2.86(6H,m), 1.95-2.00(2H,m), 1.23(3H,t)

Example 2

Synthesis of monopotassium 2-[[5-ethyl-3-[2' -(1H-tetrazol-5-yl)biphenyl-4-yl]methyl-1,3,4-thiadiazolin-2-ylidene] aminocarbonyl]-1-cyclopentenecarboxylate (compound B) To a mixture of 8.3 mL of 0.05 N potassium hydroxide (ethanolic) and 50 mL of ethanol was added 206 mg of 2-[[5-ethyl-3-[2'-(1H-tetrazol-5-yl)biphenyl-4-yl]methyl-1,3,4-thiadiazolin-2-ylidene]aminocarbonyl]-1-cyclopentenecarboxylic acid. After the mixture had been completely dissolved by heating on water bath, the solvent was evaporated in vacuo. To the residue was added ethanol. Precipitated solid was collected by filtration and dried in vacuo, whereby 180 mg of the title compound was obtained.
IR(KBr)$cm^{-1}$: 1680(-COOH) 1570(=N-CO-)

Test 1

Antihypertensive action on renal hypertensive rats (invasive)

Renal hypertensive rats were each prepared by constricting the left renal artery of a male SD rat (age: 6 weeks old, body weight: 190-220 g) with a silver clip (inner diameter: 0.017 inch) under anesthesia. Antihypertensive action was studied employing the rats whose mean blood pressure arose to 150 mmHg or higher in 4-8 weeks after the constriction of the renal artery. On the day before the test, a cannula for measure of blood pressure was inserted into the femoral artery of each of the renal hypertensive rats under anesthesia and was allowed to remain in the artery. The rats were maintained on food and water *ad libitum* until immediately before the test was started. The cannula so inserted was connected to a blood pressure transducer and a mean blood pressure was recorded on a polygraph. After the blood pressure became stable, the test compound which was suspended in 0.5% carboxymethyl cellulose was orally administered to each of the rats at 0.3 mg/kg. In accordance with the following equation, a decreased rate of blood pressure (%) was calculated from the blood pressure values before and after the administration of the test compound. The results are shown in Table 1.

$$\text{Decreased rate of blood pressure } (\%) = \frac{\text{(Blood pressure before administration of the compound} - \text{Blood pressure after administration of the compound)}}{\text{Blood pressure before administration of the compound}} \times 100$$

Table 1

| Test compound | Rate of decrease in blood pressure |
|---|---|
| Free compound (Compound(2)) | 10.8 |
| Compound A (2K salt) | 23.0 |
| Compound B (1K salt) | 19.8 |
| Control 2Na salt | 12.9 |

Test 2

Inhibitory action of the test compounds orally administered to arousal, normal-blood-pressure rats whose blood pressures had intentionally been raised by angiotensin II (intravenously injected at 0.1 µg/kg) (invasive)

On the day before the test, a cannula for the measurement of blood pressure and another cannula for the administration of a solution of angiotensin II (AII) in physiological saline (intravenously administered at 0.1 µg/kg) were inserted into the right femoral artery and the right femoral vein of each of the male SD rats (body weight: 200-350 g), respectively, under anesthesia. The rats were maintained on food and water *ad libitum* until immediately before the test was started. On the testing day, the former cannula so inserted was connected to a blood pressure transducer and the mean blood pressure was recorded on a polygraph. The solution of AII in physiological saline was then intravenously administered to each of the rats to raise its blood pressure. The blood-pressure raising response was repeated several times. After it was confirmed that the response became stable, the test compound which had been suspended in 0.5% carboxymethyl cellulose was orally administered to the rat at 1.0 mg/kg. In accordance with the following equation, an inhibitory rate (%) of blood pressure increase was calculated from the blood pressure increases before and after the administration of the test compound. The results are shown in Table 2.

$$\text{Inhibition rate of blood pressure increase (\%)} = \frac{\begin{matrix}\text{(Blood pressure increase before administration of the compound)} \\ - \\ \text{(Blood pressure increase after administration of the compound)}\end{matrix}}{\text{Blood pressure increase before administration of the compound}} \times 100$$

Table 2

| Test compound | Inhibition of blood pressure increase (%) |
|---|---|
| Free Compound (Compound (2)) | 54.2 |
| Compound (A) (2K salt) | 84.6 |

Test 3

Measurement of bioavailability in rat

SD Male rats of 6 weeks old (5 rats a group) were fasted overnight. The test compound was then administered to each of the rats at 3 mg/kg intravenously after dissolved in physiological saline and orally after suspended in 0.5% carboxymethyl cellulose, respectively. After administration, blood was collected periodically. From the blood so collected, plasma was separated by centrifugation. The test compound in the plasma was determined by high-performance liquid chromatography and based on it, areas under concentration (AUC) in intravenous administration and oral administration were found respectively. Bioavailability (BA) of the test compound for the rats was calculated in accordance with the following equation. The test results are shown in Table 3.

$$\text{BA (\%)} = \frac{\text{Area under concentration when orally administered}}{\text{Area under concentration when intravenously administered}} \times 100$$

Table 3

| Test compound | BA (%) |
|---|---|
| Free compound (Compound (2)) | 12.0 |
| Compound A (2K salt) | 42.8 |

As is apparent from the results of Tests 1, 2 and 3, the mono or dipotassium salt of the compound (2) has, compared with the compound (2) and its sodium salt, a potent angiotensin II antagonist activity and powerful antihypertensive effect and also has high bioavailability in oral administration.

**Industrial Applicability**

The mono or dipotassium salt of the biphenylmethane derivative according to the present invention has a potent angiotensin II antagonist activity and high bioavailability compared with free biphenylmethane derivatives or salts thereof other than potassium salts of biphenylmethane derivatives, so that it is not only useful as a therapeutic for circulatory

diseases such as hypertension, heart diseases and cerebral apoplexy but also stable to light and heat. It is, therefore, a very useful drug in clinical use.

## Claims

1. Mono or dipotassium 2-[[5-ethyl-3-[2'-(1H-tetrazol-5-yl)biphenyl-4-yl]methyl-1,3,4-thiadiazolin-2-yliden]aminocarbonyl]-1-cyclopentenecarboxylate.

2. A pharmaceutical composition comprising mono or dipotassium 2-[[5-ethyl-3-[2'-(1H-tetrazol-5-yl)biphenyl-4-yl]methyl-1,3,4-thiadiazolin-2-yliden]aminocarbonyl]-1-cyclopentenecarboxylate and a pharmaceutical carrier.

3. A composition according to claim 2, which is for the treatment of a circulatory disease.

4. A composition according to claim 2, which is for the treatment of hypertension.

5. A mono or dipotassium 2-[[5-ethyl-3-[2'-(1H-tetrazol-5-yl)biphenyl-4-yl]methyl-1,3,4-thiadiazolin-2-yliden]aminocarbonyl]-1-cyclopentenecarboxylate for use as a pharmaceutical.

6. The mono or dipotassium 2-[[5-ethyl-3-[2'-(1H-tetrazol-5-yl)biphenyl-4-yl]methyl-1,3,4-thiadiazolin-2yliden]aminocarbonyl]-1-cyclopentenecarboxylate for the use according to claim 5, wherein the pharmaceutical is a therapeutic of a circulatory disease.

7. The mono or dipotassium 2-[[5-ethyl-3-[2'-(1H-tetrazol-5-yl)biphenyl-4-yl]methyl-1,3,4-thiadiazolin-2-yliden]aminocarbonyl]-1-cyclopentenecarboxylate for the use according to claim 5, wherein the pharmaceutical is a therapeutic of hypertension.

## Patentansprüche

1. Mono- oder Dikalium-2-[[5-ethyl-3-[2'-(1H-tetrazol-5-yl)biphenyl-4-yl]methyl-1,3,4-thiadiazolin-2-yliden]aminocarbonyl]-1-cyclopentencarboxylat.

2. Pharmazeutische Zubereitung, umfassend das Mono-oder Dikalium-2-[[5-ethyl-3-[2'-(1H-tetrazol-5-yl)biphenyl-4-yl]methyl-1,3,4-thiadiazolin-2-yliden]aminocarbonyl]-1-cyclopentencarboxylat und einen pharmazeutischen Träger.

3. Zubereitung nach Anspruch 2, dadurch **gekennzeichnet**, daß sie zur Behandlung einer Kreislaufkrankheit verwendet wird.

4. Zubereitung nach Anspruch 2, dadurch **gekennzeichnet**, daß sie zur Behandlung von Bluthochdruck verwendet wird.

5. Mono- oder Dikalium-2-[[5-ethyl-3-[2'-(1H-tetrazol-5-yl)biphenyl-4-yl]methyl-1,3,4-thiadiazolin-2-yliden]aminocarbonyl]-1-cyclopentencarboxylat für die Verwendung als Pharmazeutikum.

6. Mono- oder Dikalium-2-[[5-ethyl-3-[2'-(1H-tetrazol-5-yl)biphenyl-4-yl]methyl-1,3,4-thiadiazolin-2-yliden]aminocarbonyl]-1-cyclopentencarboxylat für die Verwendung nach Anspruch 5, dadurch **gekennzeichnet**, daß das Pharmazeutikum ein Therapeutikum für eine Kreislaufkrankheit ist.

7. Mono- oder Dikalium-2-[[5-ethyl-3-[2'-(1H-tetrazol-5-yl)biphenyl-4-yl]methyl-1,3,4-thiadiazolin-2-yliden]aminocarbonyl]-1-cyclopentencarboxylat für die Verwendung nach Anspruch 5, dadurch **gekennzeichnet**, daß das Pharmazeutikum ein Therapeutikum für Bluthochdruck ist.

## Revendications

1. 2-{[5-Ethyl-3-[2'-(1H-tétrazol-5-yl)-biphényl-4-yl]-méthyl-1,3,4-thiadiazolin-2-ylidène]-aminocarbonyl}-1-cyclopentène carboxylate mono- ou di-potassique.

2. Composition pharmaceutique comprenant du 2-{[5-éthyl-3-[2'-(1H-tétrazol-5-yl)biphényl-4-yl)]méthyl-1,3,4-thiadiazolin-2-ylidène]-aminocarbonyl}-1-cyclopentène carboxylate mono- ou di-potassique et un support pharmaceutique.

3. Composition suivant la revendication 2, qui est pour le traitement d'une maladie vasculaire.

4. Composition suivant la revendication 2, qui est pour le traitement d'hypertension.

5. 2-{[5-Ethyl-3-[2'-(1H-tétrazol-5-yl)-biphényl-4-yl]-méthyl-1,3,4-thiadiazolin-2-ylidène]-aminocarbonyl}-1-cyclopentène carboxylate mono- ou di-potassique utile comme produit pharmaceutique.

6. 2-{[5-Ethyl-3-[2'-(1H-tétrazol-5-yl)-biphényl-4-yl]-méthyl-1,3,4-thiadiazolin-2-ylidène]-aminocarbonyl}-1-cyclopentène carboxylate mono- ou di-potassique pour l'utilisation suivant la revendication 5, dans laquelle le produit pharmaceutique est un agent thérapeutique pour une maladie vasculaire.

7. 2-{[5-Ethyl-3-[2'-(1H-tétrazol-5-yl)-biphényl-4-yl]-méthyl-1,3,4-thiadiazolin-2-ylidène]-aminocarbonyl}-1-cyclopentène carboxylate mono- ou di-potassique pour l'utilisation suivant la revendication 5, dans laquelle le produit pharmaceutique est un agent thérapeutique pour l'hypertension.